(19) Europäisches Patentamt
European Patent Office
Office européen des brevets

(11) Numéro de publication : **0 515 283 A1**

# (12) DEMANDE DE BREVET EUROPEEN

(21) Numéro de dépôt : **92401403.8**

(22) Date de dépôt : **22.05.92**

(51) Int. Cl.$^5$ : **C07H 15/12,** C07H 3/04, B01F 17/56

(30) Priorité : **22.05.91 FR 9106175**

(43) Date de publication de la demande : **25.11.92 Bulletin 92/48**

(84) Etats contractants désignés : **BE CH DE ES FR GB IT LI LU NL PT**

(71) Demandeur : **STEPAN EUROPE**
**Chemin Jonking, B.P. 127**
**F-38340 Voreppe (FR)**

(72) Inventeur : **El Ghoul, Mustapha**
**Insa-Résidence 3-no319, Avenue de Rangueil**
**F-31077 Toulouse Cedex (FR)**
Inventeur : **Rico, Isabelle**
**8 Résidence des Jardins Occitans**
**F-31520 Ramonville st Agne (FR)**
Inventeur : **Godefroy, Lionel**
**32 rue Victor Hugo**
**F-38430 Moirans (FR)**
Inventeur : **Latge, Patricia**
**25 rue du Bon Voisin, Tour III**
**F-31400 Toulouse (FR)**
Inventeur : **Lattes, Armand**
**8 Résidence des Jardins Occitans**
**F-31520 Ramonville st Agne (FR)**

(74) Mandataire : **Gillard, Marie-Louise**
**Cabinet Beau de Loménie 55, Rue d'Amsterdam**
**F-75008 Paris (FR)**

## (54) N-alkyl, N-acétylosylamines, leur procédé de préparation et leurs utilisation notamment en tant qu'agents tensioactifs ou agents de solubilisation pour l'isolement de protéines membranaires.

(57) L'invention concerne des N-alkyl, N-acétylosylamines, caractérisées en ce qu'elles répondent à la formule (I) :

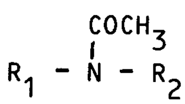

dans laquelle
— $R_1$ représente le résidu d'un sucre réducteur choisi parmi le glucose, le galactose, le lactose ou le cellobiose,
— $R_2$ est un radical alkyle, linéaire ou ramifié, comprenant de 1 à 24 atomes de carbone, de préférence de 8 à 18 atomes de carbone sous réserve que lorsque $R_1$ est un résidu de lactose, $R_2$ soit différent du radical nonyle, leur procédé de préparation et leur utilisation en tant qu'agents tensioactifs ou agents de solubilisation pour l'isolement de protéines membranaires.

EP 0 515 283 A1

L'invention concerne de nouveaux dérivés N-alkyl,N-acétylosylamines à longue chaîne, leur procédé de préparation et leurs utilisations notamment en tant qu'agents tensioactifs.

Ces nouveaux dérivés sont préparés à partir de monosaccharides ou de disaccharides réducteurs, tels que notamment le glucose, le galactose, le lactose ou le cellobiose.

En effet, le fait de greffer une longue chaîne alkyle sur ces sucres préalablement aminés permet d'obtenir des agents tensioactifs originaux à tête polaire chirale, et susceptibles d'être peu toxiques.

Néanmoins, les dérivés N-alkylosylamines ne peuvent être utilisés directement comme agents tensioactifs car ils sont instables en solution aqueuse. De manière inattendue, on a maintenant trouvé que l'acylation des dérivés N-alkylosylamines améliorait leur stabilité en solution aqueuse tout en conservant leurs propriétés tensioactives.

Des dérivés N-glycosylés d'amides d'acides carboxyliques sont décrits dans les demandes de brevets EP 0 091 645, 0 147 777 et 0 140 321.

La demande EP 0 091 645 décrit des dérivés à activité immunostimulante de formule

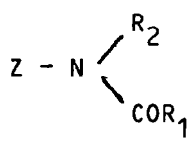

dans laquelle

- Z est un monosaccharide ;
- $R_1$ est notamment un alkyle à longue chaîne en $C_9$-$C_{21}$ ;
- $R_2$ est un aralkyle ou un alkyle comportant jusqu'à 30 atomes de carbone.

Les demandes EP 0 147 777 et 0 140 321 concernent l'utilisation, respectivement, en tant qu'agents à activité anti-rhumatismale, ou agents favorisant la croissance dans l'alimentation animale, de composés de formule

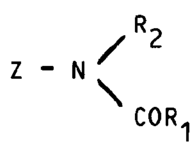

dans laquelle

- Z est un monosaccharide ;
- $R_1$ est l'hydrogène ou un radical hydrocarboné en $C_1$-$C_{30}$ .
- $R_2$ est l'hydrogène ou un radical alkyle ou aralkyle ayant jusqu'à 30 atomes de carbone, sous réserve que $COER_1$ ne soit pas un radical en $C_1$-$C_5$ si $R_2$ est un alkyle en $C_{10}$-$C_{20}$.

Un dérivé N-dodécyl-N-β-D glycopyranosyl-acétamide est notamment décrit dans ces demandes.Néanmoins, le procédé d'obtention décrit, à savoir l'acétylation par l'anhydride acétique dans la pyridine résultant en une acétylation totale suivie d'une O-déacétylation, ne permet pas d'obtenir ce produit sous forme pure mais sous forme d'un mélange de produits O- et N-acétylés.

L'invention a donc pour objet des dérivés N-alkyl,N-acétylosylamines de formule (I) :

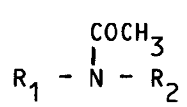

(I)

dans laquelle :

- $R_1$ représente le résidu d'un sucre réducteur choisi parmi le glucose, le galactose, le lactose ou le cellobiose,
- $R_2$ est un radical alkyle, linéaire ou ramifié; comprenant de 1 à 24 atomes de carbone.

Les dérivés de formule (I), sous réserve que, lorsque $R_1$ est un résidu de lactose, $R_2$ soit différent d'un radical nonyle, sont des produits nouveaux.

Avantageusement l'invention concerne des dérivés de formule (I) dans laquelle $R_1$ est le résidu d'un glucose, c'est-à-dire les dérivés de formule (II) :

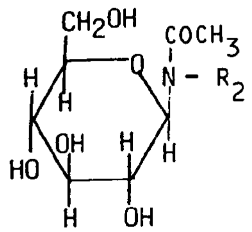

(II)

dans laquelle $R_2$ est un radical alkyle, linéaire ou ramifié, comprenant de 1 à 24 atomes de carbone.

D'autres dérivés avantageux sont les dérivés de formule (I) dans laquelle $R_1$ est le résidu d'un lactose, tels que représentés par la formule (III) :

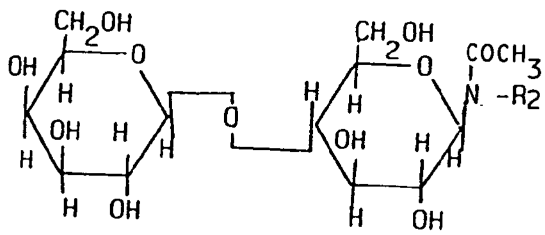

(III)

dans laquelle $R_2$ est un radical alkyle, linéaire ou ramifié, comprenant de 1 à 24 atomes de carbone, à l'exception du radical nonyle.

Les dérivés de formules (I), (II) et (III) dans lesquelles $R_2$ est un radical alkyle, linéaire ou ramifié, comprenant de 8 à 18 atomes de carbone sous réserve que lorsque $R_1$ est un résidu de lactose, $R_2$ soit différent d'un radical nonyle, sont particulièrement préférés.

Les dérivés de formules (I), (II) ou (III) dans lesquelles $R_2$ est un radical alkyle, linéaire ou ramifié, comprenant de 10 à 20 atomes de carbone, représentent un autre aspect avantageux de l'invention.

Les N-alkyl,N-acétylosylamines de formule (I) peuvent être préparées par acylation des N-alkylosylamines correspondantes de formule (IV) :

$$R_1 - NH - R_2 \qquad (IV)$$

dans laquelle :

- $R_1$ représente le résidu d'un sucre réducteur choisi parmi le glucose, le galactose, le lactose ou le cellobiose,
- $R_2$ est un radical alkyle, linéaire ou ramifié, comprenant de 1 à 24 atomes de carbone.

Les N-alkylosylamines de formule (IV) peuvent elles-mêmes être préparées selon la méthode de J.G. ERIKSON (J. of Am. Chem. Soc., 77, 2839 (1955)) qui consiste à faire réagir le sucre avec une amine à longue chaîne $RNH_2$ dans un mélange d'éthanol et d'eau laissé à 25°C pendant 24 heures. On obtient les N-alkylosylamines par simple filtration. Le précipité formé est recristallisé dans l'éthanol puis lyophilisé.

Dans une seconde étape, les N-alkylosylamines de formule (IV) sont soumises à une acylation.

Cette étape d'acylation consiste à faire réagir la N-alkylosylamine de formule (IV) avec de l'anhydride acétique dans un solvant aprotique apolaire, tel que par exemple le diméthylformamide (DMF) ou le diméthylsulfoxyde (DMSO), sous atmosphère inerte, par exemple sous atmosphère d'azote ou d'argon, à température ambiante, pendant une durée de 12 à 48 heures, de préférence 24 heures.

Les N-alkyl,N-acétylosylamines ainsi obtenues de formule (I), se présentent sous forme de solides blancs, largement solubles et stables en solution aqueuse.

Les caractéristiques RMN, les spectres de masse et les microanalyses confirment leur structure correspondant à la formule (I).

Ces dérivés peuvent être utilisés comme agents tensioactifs. Ils peuvent également présenter des propriétés intéressantes comme agents de solubilisation de protéines pour l'isolement de protéines membranaires, ou encore comme inducteurs d'asymétrie dans des systèmes micellaires.

Les exemples suivants illustrent l'invention sans la limiter.

EXEMPLE 1 : N-heptyl,N-acétylglucosylamine

a - Préparation de la N-heptylglucosylamine.

A une solution contenant 9 g de glucose et 35 ml d'eau, on ajoute une solution d'heptylamine (11,5 g dans 50 ml d'éthanol). Le mélange réactionnel est agité pendant 24 heures à température ambiante. Le précipité formé est filtré et recristallisé dans l'éthanol, puis lyophilisé.

On obtient 9 g de produit solide blanc dont les analyses montrent qu'il s'agit du composé de formule :

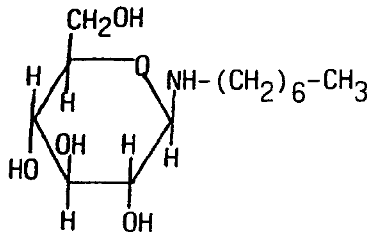

b - Préparation de la N-heptyl,N-acétylglucosylamine.

A une solution de 3,5 g de N-heptylglucosylamine dans 90 ml de DMF, sous argon, 1,3 g de triéthylamine et 1,3 g d'anhydride acétique sont ajoutés, à température ambiante. Le mélange réactionnel est laissé sous agitation pendant 12 heures. Le solvant est évaporé sous vide. Le produit est lavé à l'eau, lyophilisé et purifié sur colonne de silice en éluant avec un mélange chloroforme/méthanol/ammoniaque (10:5:0,5).

On obtient 1,5 g de produit solide dont les analyses montrent qu'il s'agit du composé de formule :

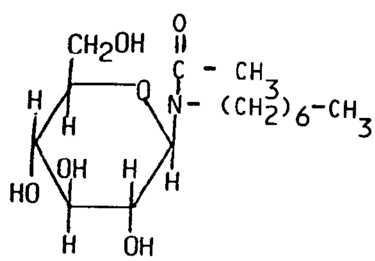

EXEMPLE 2 : N-octyl,N-acétylglucosylamine

a - Préparation de la N-octylglucosylamine.

On procède de la même façon que dans l'exemple 1 en utilisant 9 g de glucose et 13,2 g d'octylamine.

On récupère 6,5 g de produit solide blanc dont les analyses montrent qu'il s'agit du produit de formule :

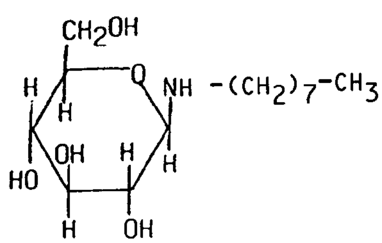

b - Préparation de la N-octyl,N-acétylglucosylamine.

On utilise le même protocole expérimental que dans l'exemple 1 en utilisant 4 g de N-octylglucosylamine, 1,4 g de triéthylamine, 1,4 g d'anhydride acétique et 100 ml de DMF.

On obtient 1 g de produit solide dont les analyses montrent qu'il s'agit du composé de formule :

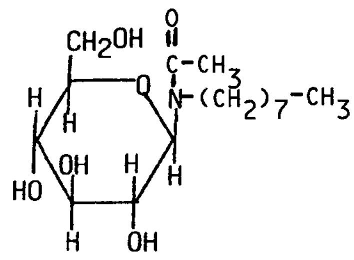

EXEMPLE 3 : N-nonyl,N-acétylglucosylamine

a - Préparation de la N-nonylglucosylamine.

On procède de la même façon que dans les exemples précédents en utilisant 9 g de glucose et 14,3 g de nonylamine.

On récupère 8,8 g de produit solide blanc dont les analyses montrent qu'il s'agit du produit de formule :

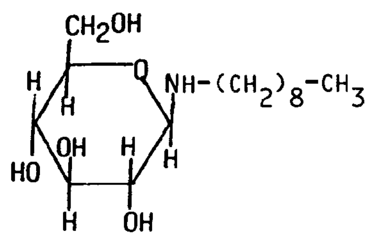

b - Préparation de la N-nonyl,N-acétylglucosylamine.

On procède de la même façon que dans les exemples précédents en utilisant 3,4 g de N-nonylglucosylamine, 1,1 g de triéthylamine, 1,1 g d'anhydride acétique et 80 ml de DMF.

On obtient 1 g de produit solide dont les analyses montrent qu'il s'agit du composé de formule :

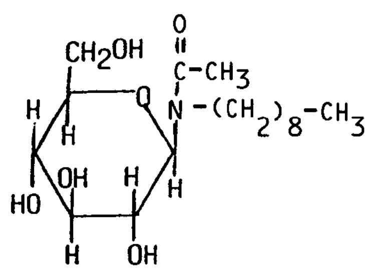

EXEMPLE 4 : N-décyl,N-acétylglucosylamine

a - Préparation de la N-décylglucosylamine.

On procède de la même façon que dans les exemples précédents en utilisant 9 g de glucose et 15,7 g de décylamine.

On récupère 7,5 g de produit de solide blanc dont les analyses montrent qu'il s'agit du produit de formule :

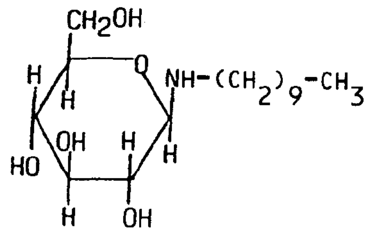

b - Préparation de la N-décyl,N-acétylglucosylamine.

On procède de la même façon que dans les exemples précédents en utilisant 4 g de N-décylglucosylamine, 1,3 g de triéthylamine, 1,3 g d'anhydride acétique et 100 ml de DMF.

On obtient 2,5 g de produit solide dont les analyses montrent qu'il s'agit du composé de formule :

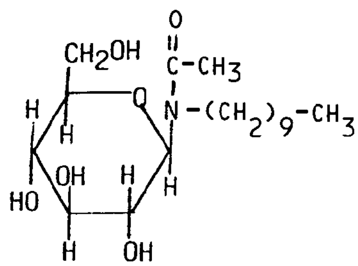

EXEMPLE 5 : N-nonyl,N-acétyllactosylamine

a - Préparation de la N-nonyllactosylamine.

A une solution contenant 21 g de lactose et 120 ml d'eau, on ajoute une solution de nonylamine (14,1 g) dans 200 ml de propanol-2. Le mélange réactionnel est agité 24 heures à température ambiante puis chauffé à 60°C pendant 30 min.

Le solvant est évaporé sous pression réduite et le résidu est repris à l'éthanol puis réévaporé en présence de toluène pour éliminer l'eau résiduelle. Le solide final est recristallisé dans l'éthanol puis lyophilisé.

On obtient 17,6 g de produit solide blanc sont les analyses montrent qu'il s'agit du composé de formule :

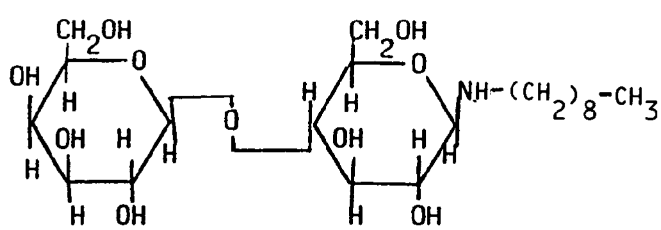

b - Préparation de la N-nonyl,N-acétyllactosylamine

A une solution de 6 g de N-nonyllactosylamine dans 80 ml de DMF, sous argon, 1,3 g de triéthylamine et 1,3 g d'anhydride acétique sont ajoutés à température ambiante. Le mélange réactionnel est laissé sous agitation pendant 12 heures. Le solvant est évaporé sous vide. Le produit est lavé à l'eau, lyophilisé est purifié sur colonne de silice en éluant avec un mélange chloroforme/méthanol/ammoniaque (20:15:2).

On obtient 3,4 g de produit solide dont les analyses montrent qu'il s'agit du composé de formule :

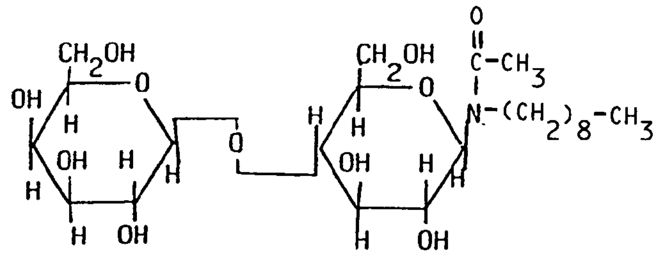

EXEMPLE 6 : N-décyl,N-acétyllactosylamine

a - Préparation de la N-décyllactosylamine.

On procède de la même façon que dans l'étape a) de l'exemple 5 en utilisant 21 g de lactose et 15,2 g de décylamine . On récupère 17,8 g de produit solide blanc dont les analyses montrent qu'il s'agit du produit de formule :

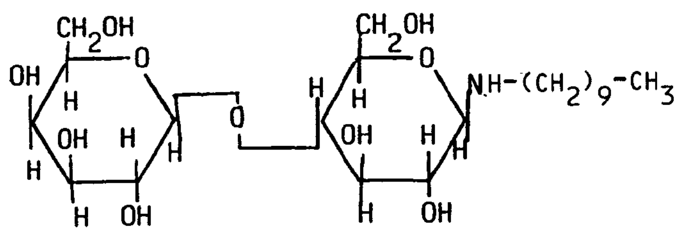

b - Préparation de la N-décyl,N-acétyllactosylamine

On procède de la même façon que dans l'étape b) de l'exemple 5 en utilisant 10 g de N-décyllactosylamine, 2,1 g de triéthylamine, 2,1 g d'anhydride acétique et 130 ml de DMF.

On obtient 4 g de produit solide dont les analyses montrent qu'il s'agit du composé de formule :

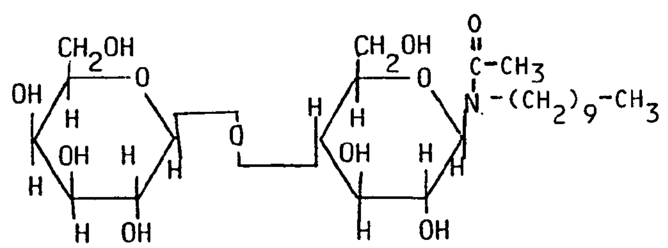

EXEMPLE 7 : N-octyl,N-acétyllactosylamine

a - Préparation de la N-octyllactosylamine.

On procède de la même façon que dans les exemples 5 et 6 précédents en utilisant 21 g de lactose et 12,8 g d'octylamine.

On récupére 16 g de produit solide blanc dont les analyses montrent qu'il s'agit du produit de formule :

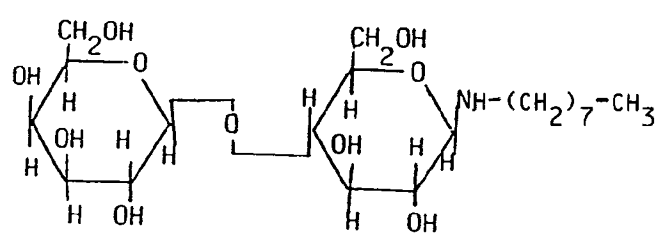

b - Préparation de la N-octyl,N-acétyllactosylamine

On procède de la même façon que dans les exemples 5 et 6 précédents en utilisant 8 g d'octyllactosylamine, 1,8 g de triéthylamine, 1,8 g d'anhydride acétique et 110 ml de DMF.

On obtient 3 g de produit solide dont les analyses montrent qu'il s'agit du composé de formule :

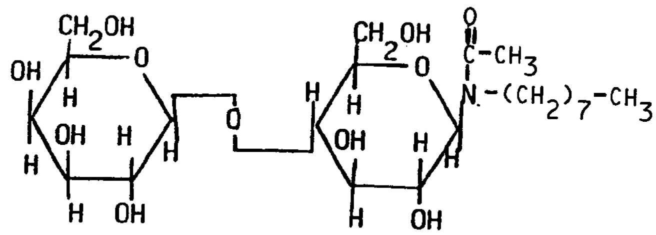

EXEMPLE 8

Les propriétés tensioactives de la N-décyl,N-acétylglucosylamine préparée selon l'exemple 4 peuvent être mesurées par une technique usuelle de tensiométrie en utilisant un tensiomètre Tensimat N3 (Prolabo, FRANCE). Les mesures sont effectuées à 25°C. Le mobile de mesure est un étrier fourni avec l'appareil formé avec un fil de platine de 0,1 mm de diamètre et long de 2 cm (écart entre les 2 branches de l'étrier).

Les résultats sont illustrés par la courbe donnée sur la figure unique dans laquelle figurent en abscisse le log de la concentration de produit et en ordonnée la tension superficielle exprimée en mN/m.

Cette courbe permet de déterminer la concentration micellaire critique (CMC) de la N-décyl,N-acétylglucosylamine qui est égale à $1{,}29.10^{-3}$ M/L.

**Revendications**

1. N-alkyl, N-acétylosylamines, caractérisées en ce qu'elles répondent à la formule (I) :

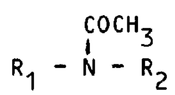

(I)

dans laquelle
- $R_1$ représente le résidu d'un sucre réducteur choisi parmi le glucose, le galactose, le lactose ou le cellobiose,
- $R_2$ est un radical alkyle, linéaire ou ramifié, comprenant de 1 à 24 atomes de carbone, sous réserve que lorsque $R_1$ est un résidu de lactose, $R_2$ soit différent du radical nonyle.

2. N-alkyl, N-acétylosylamines selon la revendication 1, caractérisées en ce que $R_2$ est un radical alkyle, linéaire ou ramifié, comprenant de 10 à 20 atomes de carbone.

3. N-alkyl, N-acétylosylamines selon la revendication 1, caractérisées en ce que $R_2$ est un radical alkyle, linéaire ou ramifié, comprenant de 8 à 18 atomes de carbone.

4. N-alkyl,N-acétylosylamines selon l'une quelconque des revendications 1 à 3, caractérisées en ce qu'elles répondent à la formule (II) :

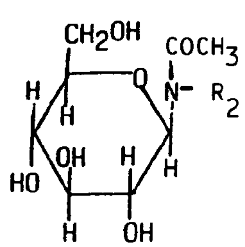

(II)

dans laquelle
$R_2$ est un radical alkyle, linéaire ou ramifié, comprenant de 1 à 24 atomes de carbone.

5. N-alkyl,N-acétylosylamines selon l'une quelconque des revendications 1 à 3 ,caractérisées en ce qu'elles répondent à la formule (II) :

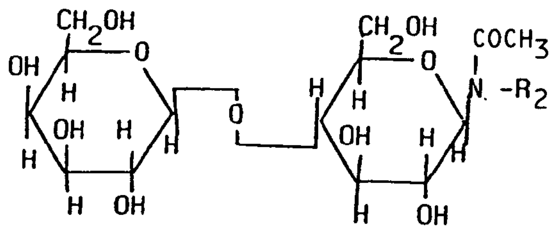
(III)

dans laquelle
$R_2$ est un radical alkyle, linéaire ou ramifié, comprenant de 1 à 24 atomes de carbone, à l'exception du radical nonyle.

6. Procédé de préparation des N-alkyl,N-acétylosylamines de formule (I) :

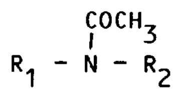
(I)

dans laquelle
- $R_1$ représente le résidu d'un sucre réducteur choisi parmi le glucose, le galactose, le lactose ou le cellobiose,
- $R_2$ est un radical alkyle, linéaire ou ramifié, comprenant de 1 à 24 atomes de carbone, caractérisé en ce que l'on soumet une N-alkylosylamine de formule (IV) :

$$R_1 - NH - R_2 \qquad (IV)$$

dans laquelle
$R_1$ et $R_2$ sont tels que définis ci-dessus à une acylation par de l'anhydride acétique dans un solvant aprotique apolaire, sous atmosphère inerte, à température ambiante, pendant une durée de 12 à 48 h.

7. Utilisation des N-alkyl, N-acétylosylamines de formule (I) :

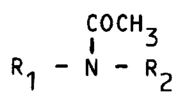
(I)

dans laquelle
- $R_1$ représente le résidu d'un sucre réducteur choisi parmi le glucose, le galactose, le lactose ou le cellobiose,
- $R_2$ est un radical alkyle, linéaire ou ramifié, comprenant de 1 à 24 atomes de carbone, en tant qu'agents tensioactifs.

8. Utilisation des N-alkyl, N-acétylosylamines de formule (I) :

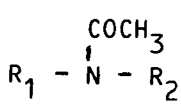
(I)

dans laquelle
- $R_1$ représente le résidu d'un sucre réducteur choisi parmi le glucose, le galactose, le lactose ou le cellobiose,

- $R_2$ est un radical alkyle, linéaire ou ramifié, comprenant de 1 à 24 atomes de carbone, en tant qu'agents de solubilisation pour l'isolement de protéines membranaires.

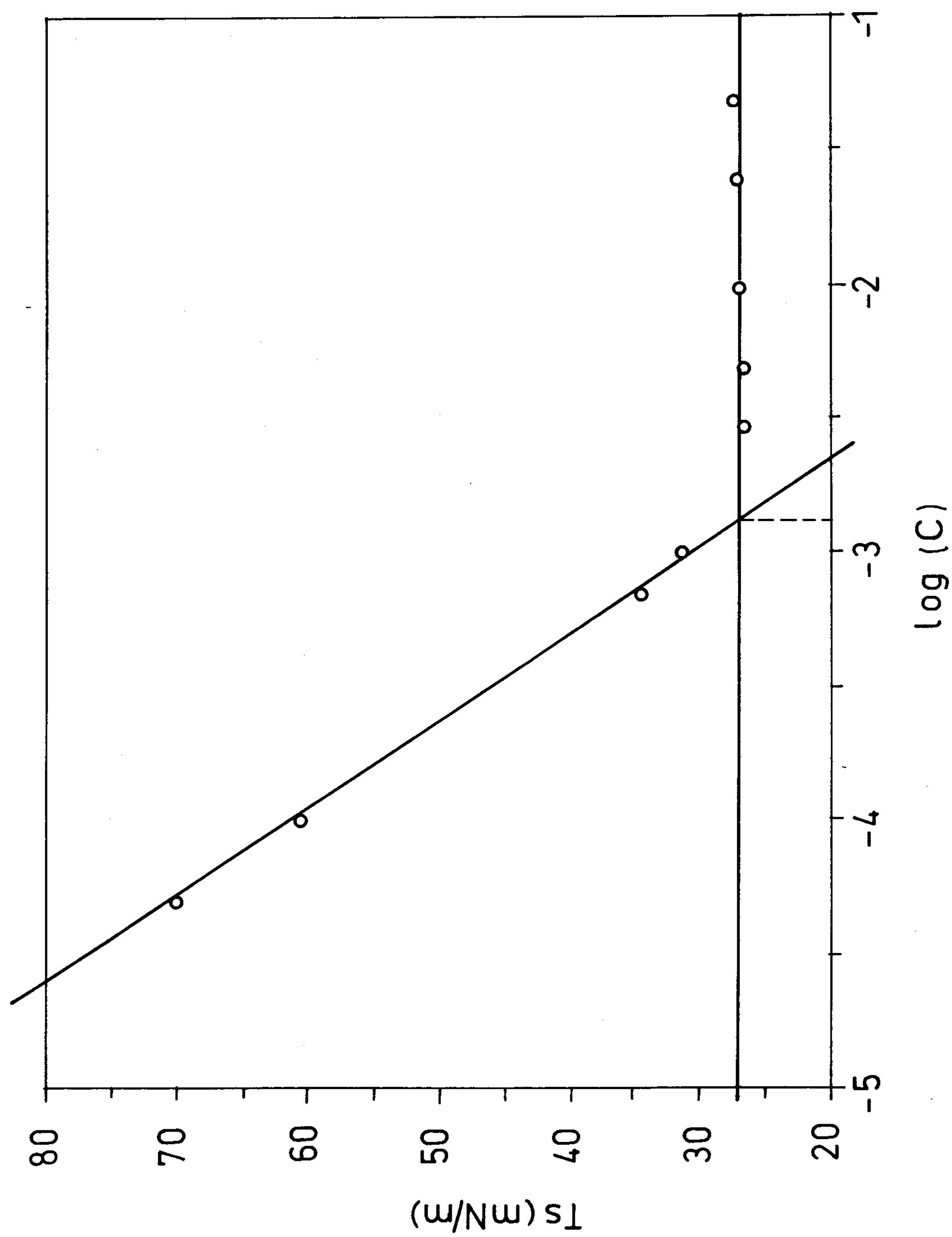
Ts (mN/m)
80
70
60
50
40
30
20
log (C)
−5
−4
−3
−2
−1

Office européen des brevets

# RAPPORT DE RECHERCHE EUROPEENNE

Numero de la demande

EP 92 40 1403

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5 ) |
|---|---|---|---|
| X | EP-A-0 091 645 (BAYER AG)<br>* page 5; revendications 1,7; exemples 3,4 *<br>--- | 1-6 | C07H15/12<br>C07H3/04<br>B01F17/56 |
| X | EP-A-0 147 777 (BAYER AG)<br>* page 1 - page 5; revendication 1; exemples 4,5 *<br>--- | 1-6 | |
| X | EP-A-0 140 321 (BAYER AG)<br>* page 5; exemples 4,5 *<br>--- | 1-6 | |
| X | CHEMICAL ABSTRACTS, vol. 87, no. 25, 19 Décembre 1977, Columbus, Ohio, US; abstract no. 201958,<br>MASCIORINI E; THIEL I M E; DEFERRARI J O 'Synthesis of some N-methyl-N-acyl-aldopyranosylamines and their per-O-acyl derivatives'<br>page 768 ;colonne 1 ;<br>* abrégé *<br>& AN. QUIM<br>vol. 72, no. 11, 1976,<br>pages 946 - 949<br>--- | 1 | **DOMAINES TECHNIQUES RECHERCHES (Int. Cl.5 )**<br>C07H |
| P,X | FR-A-2 657 611 (ECOLE NATIONALE SUPERIEURE DE CHEMIE DE RENNES (E.P.C.A.))<br>* page 1; revendications *<br>--- | 1-5,7,8 | |
| P,X | FR-A-2 661 413 (STEPAN EUROPE)<br>* page 9 - page 11; figure 1 *<br>--- | 1-8 | |
| A | WO-A-8 706 236 (RESEARCH CORPORATION LIMITED)<br>* revendications 1,9; exemple 2 *<br>----- | 8 | |

Le présent rapport a été établi pour toutes les revendications

| Lieu de la recherche | Date d'achèvement de la recherche | Examinateur |
|---|---|---|
| LA HAYE | 11 SEPTEMBRE 1992 | DAY Graham James |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)